# EUROPEAN PATENT APPLICATION

(11) **EP 4 321 534 A1**
(43) Date of publication of application: **14.02.2024**
(21) Application number: 22784700.1
(22) Date of filing: 07.04.2022
(51) Int. Cl.: C07K 16/28, C12N 5/0783, C12N 15/12, C12N 15/13, C12N 15/62

(54) **METHOD FOR ACTIVATING T-CELLS**

(30) Priority: 08.04.2021 JP 2021066050
(71) Applicant: Takeda Pharmaceutical Company Limited, Osaka-shi, Osaka 541-0045 (JP); Noile-Immune Biotech Inc., Tokyo 105-0012 (JP)
(72) Inventor: OGAKI, Soichiro, Fujisawa-shi, Kanagawa 251-0012 (JP); ARAKI, Hideo, Fujisawa-shi, Kanagawa 251-0012 (JP); MAEDA, Eiki, Fujisawa-shi, Kanagawa 251-0012 (JP)
(74) Representative: Jones, Nicholas Andrew
(86) International application number: PCT/JP2022/017224
(87) International publication number: WO 2022/215718

(57) **Abstract**

Provided is a method for activating T cells comprising the step of activating T cells in a medium containing a CD3 agonist and/or a CD28 agonist for more than 36 hours and less than 48 hours; a method for producing genetically modified T cells, comprising the steps of activating T cells according to the activation method, introducing an exogenous gene into the activated T cells, and culturing the T cells into which the exogenous gene has been introduced; a cell population comprising genetically modified T cells obtained by the production method; and a matrix of mobile polymer chains or a bead supporting a CD3 agonist and/or a CD28 agonist, wherein the matrix or bead is used for being added to a medium and subjecting T cells to an activation step for more than 36 hours and less than 48 hours.

## Description

### Technical Field

The present invention relates to a method for activating T cells. More specifically, the present invention relates to a method for activating T cells that is effective for producing T cells (referred to as "CAR-T cells" in this specification) that express a chimeric antigen receptor (referred to as "CAR" in this specification).

### Background of the Invention

CAR-T cell therapy is known as one of the methods to treat cancer. CAR-T cells are, for example, produced using autologous T cells collected from patients themselves; however, high manufacturing costs and the risk of manufacturing failure are problems in running business. In particular, since as compared to healthy people, many cancer patients who receive CAR-T cell therapy have damaged T cells due to treatment with an anticancer drug and the like, the production efficiency of CAR-T cells from patient-derived T cells tends to be reduced.

To produce CAR-T cells, the step of activating T cells using stimulatory substances (e.g., an anti-CD3 antibody and an anti-CD28 antibody) is typically performed. For example, Patent Literature 1 discloses that a matrix (nanomatrix) of mobile polymer chains to which a stimulant substance is attached is used for the activation of T cells.

In general, the T-cell activation step has been performed for multiples of one day, such as 24 hours, 48 hours (2 days), or 72 hours (3 days) (Non-patent Literature 1 to 7).

### Citation List

### Patent Literature

PTL 1: WO2014/048920

### Non-patent Literature

NPL 1: MACS (trademark) GMP T Cell TransAct (trademark) Manual
NPL 2: Fernandez et al., Front. Immunol. 2019, 10:2361.
NPL 3: Aleksandrova et al., Transfus Med Hemother 2019; 46:47-54
NPL 4: Vedvyas et al., Scientific Reports (2019) 9:10634
NPL 5: Mock et al., Cytotherapy, 2016; 18: 1002-1011
NPL 6: Blaeschke et al., Cancer Immunology, Immunotherapy (2018) 67:1053-1066
NPL 7: Arcangeli et al., Front. Immunol. 2020, 11:1217.

### Summary of Invention

### Technical Problem

An object of the present invention is to provide a means for improving the production efficiency of CAR-T cells.

### Solution to Problem

The present inventors focused on the T-cell activation step and examined T cells from multiple donors. As a result, they found that the CAR expression efficiency is improved by setting the activation duration in the T-cell activation step to a specific range (e.g., longer than 36 hours and shorter than 48 hours).

The present invention comprises at least the following inventions.
[1] A method for activating T cells comprising the step of activating T cells in a medium containing a CD3 agonist and/or a CD28 agonist for more than 36 hours and less than 48 hours.
[2] The activation method according to Item 1, wherein the CD3 agonist and/or CD28 agonist is supported on a carrier.
[3] The activation method according to Item 2, wherein the carrier is a matrix of mobile polymer chains or a bead.
[4] The activation method according to any one of Items 1 to 3, wherein the CD3 agonist and the CD28 agonist are an anti-CD3 antibody and an anti-CD28 antibody, respectively.
[5] A method for producing genetically modified T cells, comprising the steps of:
   activating T cells according to the activation method of Item 1,
   introducing an exogenous gene into the activated T cells, and
   culturing the T cells into which the exogenous gene has been introduced.
[6] A cell population comprising genetically modified T cells obtained by the production method according to Item 5.
[7] The production method according to Item 5, wherein the exogenous gene is a chimeric antigen receptor gene.
[8] A matrix of mobile polymer chains or a bead supporting a CD3 agonist and/or a CD28 agonist, wherein the matrix or bead is used for being added to a medium and subjecting T cells to an activation step for more than 36 hours and less than 48 hours.
[9] A T-cell activation kit for introducing an exogenous gene, comprising
   a CD3 agonist and/or a CD28 agonist supported on a carrier, and
   a manual describing the activation of T cells in a medium comprising the CD3 agonist and/or the CD28 agonist for more than 36 hours and less than 48 hours.

In this specification, the method for activating T cells of Item 1 or the like, the method for producing genetically modified T cells of Item 5 or the like, and the cell population comprising genetically modified T cells of Item 6 or the like are respectively referred to as the "activation method of the present invention," the "production method of the present invention," and the "cell population of the present invention."

### Advantageous Effects of Invention

Since the present invention can optimize the activation step, which places a heavy burden on T cells, and can efficiently produce genetically modified T cells such as CAR-T cells, production costs can be reduced due to the reduction in the number of production days and the production efficiency can be improved in the production of genetically modified T cells.

### Brief Description of Drawings

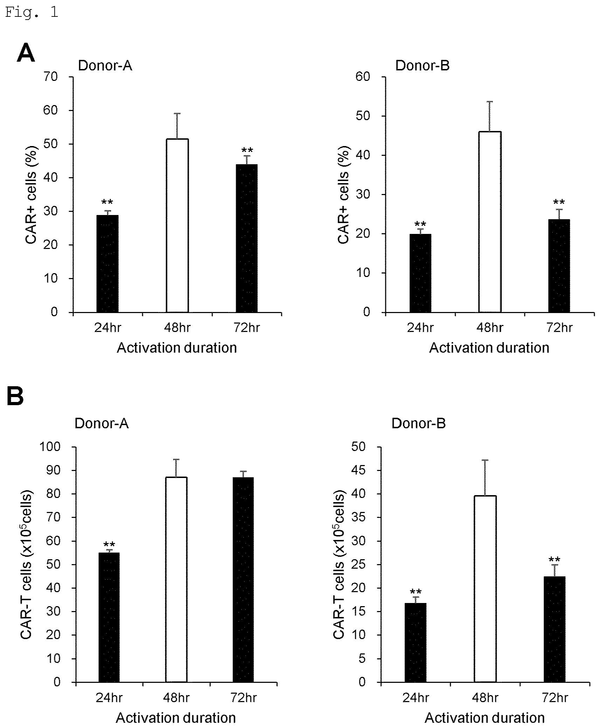

Fig. 1 shows the results in which CAR-expressing cells in cell populations (activation step of 24, 48, or 72 hours) obtained according to "(2) Production of CAR-T Cells on Small Scale Using PBMC" in the Examples are measured by "(4) Flow Cytometry". Figs. 1A and 1B show the results of the percentage of CAR-expressing cells (Fig. 1A) and the number of CAR-expressing cells (Fig. 1B) obtained using PBMC derived from two donors (Donor A and Donor B). Figs. 1A and 1B show a significant difference of **p <0.02 at 48 hours as compared to 24 hours and 72 hours (t-test, N = 3, values in the figures are mean ± standard deviation).

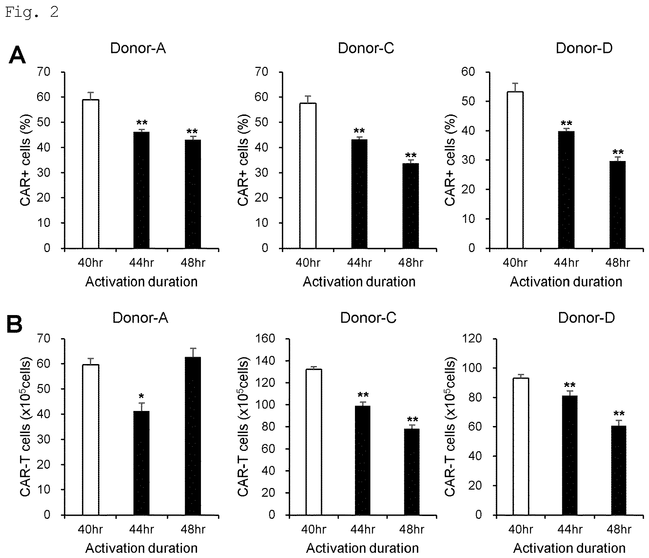

Fig. 2 shows the results in which CAR-expressing cells in cell populations (activation step of 40, 44, or 48 hours) obtained according to "(2) Production of CAR-T Cells on Small Scale Using PBMC" in the Examples are measured by "(4) Flow Cytometry". Figs. 2A and 2B show the results of the percentage of CAR-expressing cells (Fig. 2A) and the number of CAR-expressing cells (Fig. 2B) obtained using PBMC derived from three donors (Donor A, Donor C, and Donor D). Figs. 2A and 2B show a significant difference of *p<0.05 or **P<0.02 at 40 hours as compared to 44 hours and 48 hours (t-test, N = 3-6, values in the figures are mean ± standard deviation).

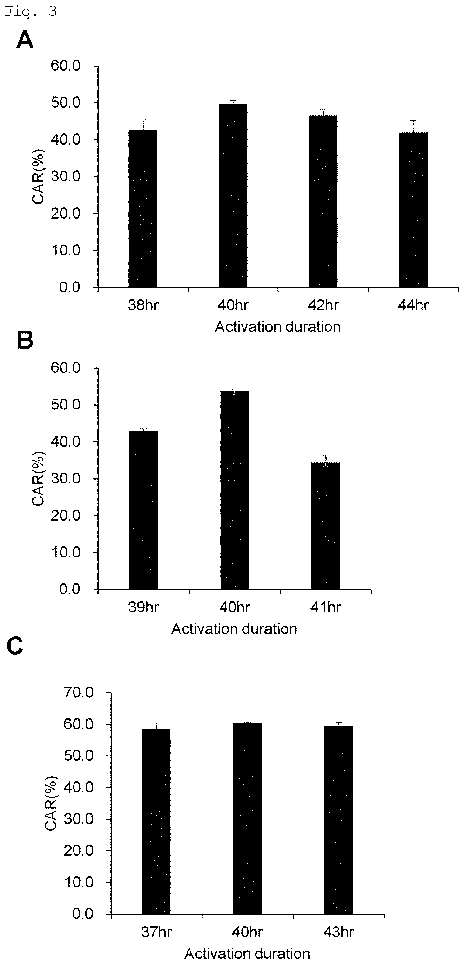

Fig. 3 shows the results in which CAR-expressing cells in cell populations (activation step of 37 to 44 hours) obtained according to "(2) Production of CAR-T Cells on Small Scale Using PBMC" in the Examples are measured by "(4) Flow Cytometry". Figs. 3A to 3C show the results of the percentage of CAR-expressing cells at each activation duration using PBMC derived from a single donor (N = 3, values in the figures are mean ± standard deviation).

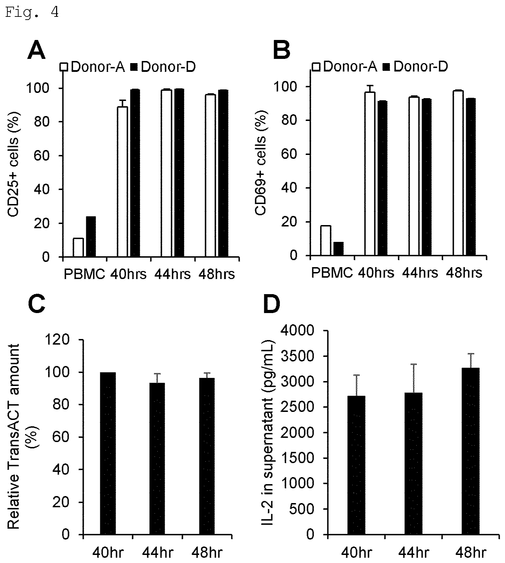

Fig. 4 shows the results in which activated T cells in cell populations (activation step of 40, 44, or 48 hours) obtained according to "(2) Production of CAR-T Cells on Small Scale Using PBMC" in the Examples are measured by "(4) Flow Cytometry" using an activation marker CD25 or CD69 as an index, and the results in which the residual amounts of TransACT and IL-2 used for activation in media are analyzed according to "(5) Analysis of Residual Amounts of TransACT and IL-2 Used for Activation." Figs. 4A and 4B show the percentage of CD25-expressing cells (Fig. 4A) and the percentage of CD69-expressing cells (Fig. 4B) obtained using PBMC derived from two donors (Donor A and Donor D) (N = 3, values in the figures are mean ± standard deviation). Figs. 4C and 4D show the amount of TransACT (Fig. 4C) remaining in the medium immediately after the activation step and the amount of IL-2 (Fig. 4D) remaining in the medium immediately after the activation step at each activation duration (40, 44, or 48 hours) obtained using PBMC derived from a single donor (N = 3, values in the figure are mean ± standard deviation).

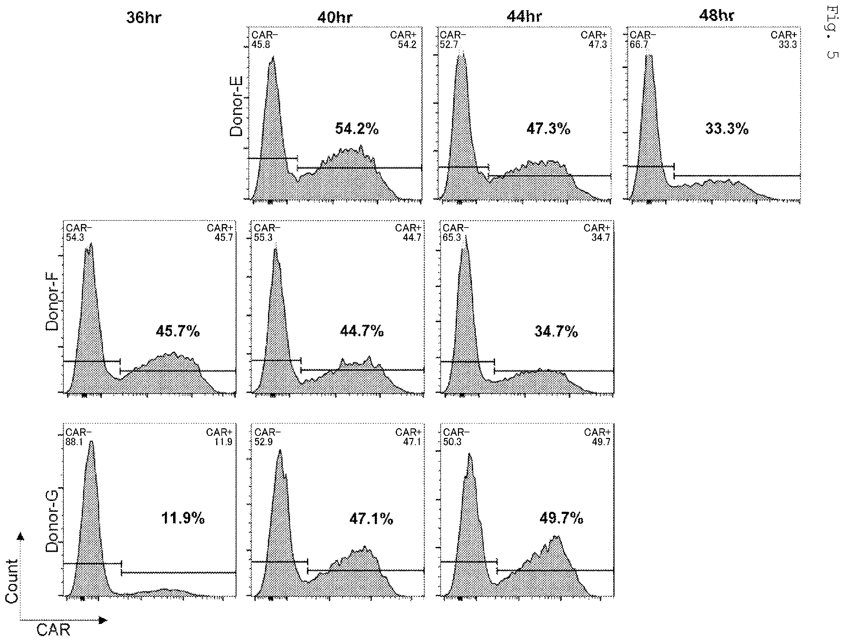

Fig. 5 shows the results in which CAR-expressing cells in cell populations (activation step of 36, 40, 44, or 48 hours) obtained according to "(3) Production of CAR-T Cells on Clinical Scale Using T Cells" in the Examples are measured by "(4) Flow Cytometry" using human leukocyte apheresis products derived from 3 donors (Donor E, Donor F, and Donor G).

### Description of Embodiments

### Method for Activating T Cells

The activation method of the present invention is a method for activating T cells comprising the step of activating T cells in a medium containing a CD3 agonist and/or a CD28 agonist for more than 36 hours and less than 48 hours (referred to as the "activation step" in this specification).

Any "T cells" can be used as long as T cells are activated according to the purpose and application when used. Examples include αβ T cells, γδ T cells, CD8⁺ T cells, CD4⁺ T cells, tumor-infiltrating T cells, memory T cells, naive T cells, NKT cells, and regulatory T cells.

T cells can generally be collected from immune cells infiltrating body fluids such as blood and bone marrow fluid; tissues such as the spleen, thymus, lymph node, and liver; or cancerous tissues such as primary tumors, metastatic tumors, and cancerous ascites; as peripheral blood mononuclear cells (PBMC) and leukocyte apheresis. The T cells used in the present invention may be specific T cells isolated and purified from a population of collected immune cells, or may be T cells contained in a cell population (e.g., PBMC) without isolation. The T cells may be those obtained by culturing induced pluripotent stem cells (iPS cells), embryonic stem cells (ES cells), or other stem or progenitor cells under appropriate conditions to induce their differentiation into T cells.

The T cells may be derived from humans or mammals other than humans (non-human mammals). Examples of non-human mammals include mice, rats, hamsters, guinea pigs, rabbits, dogs, cats, pigs, cows, horses, sheep, and monkeys.

In one embodiment of the present invention, T cells activated by the method of the present invention are T cells for introducing an exogenous gene, for example, T cells for introducing an exogenous gene for expressing a chimeric antigen receptor (CAR). In this embodiment, T cells may be, for example, CD8⁺ T cells or CD4⁺ T cells, those taken from blood, e.g., those contained in peripheral blood mononuclear cells (PBMC), or those derived from a human, e.g., a cancer patient or a human to whom CAR-T cells are to be administered.

The "CD3 agonist" and "CD28 agonist" respectively bind to CD3 and CD28 (receptors) as an agonist and are substances that can stimulate T cells for activation. A typical CD3 agonist is an anti-CD3 antibody, and a typical CD28 agonist is an anti-CD28 antibody. Substances other than the antibodies that have similar effects (e.g., T-CEP (T-cell expansion protein (Matus et al., JCI Insight, 2020, vol. 5, issue 22, 1-14) and PHA (phytohemagglutinin (Weng et al., J. Ethnopharmacol, 2002, 83(1-2) :79-85)) can also be used as CD3 and CD28 agonists. Anti-CD3 and anti-CD28 antibodies may be monoclonal or polyclonal antibodies, and are preferably monoclonal antibodies. The anti-CD3 and anti-CD28 antibodies may be fragmented. In the T-cell activation step, RetroNectin (trademark) (Recombinant Human Fibronectin Fragment, Takara Bio Inc.) may be used together with CD3 and/or CD28 agonists.

The concentrations of the CD3 agonist and the CD28 agonist are not particularly limited as long as the concentration is sufficient to stimulate the surface molecules of T cells to transmit signals into T cells and induce activation. For example, when the anti-CD3 antibody and/or the anti-CD28 antibody is used, it can be used in such an amount that the final concentration of each antibody is 0.1 to 20 pg/mL. When a matrix of mobile polymer chains (e.g., TransACT) supporting the anti-CD3 antibody and anti-CD28 antibody is used, it is used so that the final concentration of each antibody is 10 to 300 ng/mL. The molar ratio of the anti-CD3 antibody to the anti-CD28 antibody can be 1:5 to 5:1 and preferably 1:2 to 2:1.

In the activation method of the present invention, the T cells are activated by culturing them in a medium containing a CD3 agonist and/or a CD28 agonist for more than 36 hours and less than 48 hours. The activation duration of "more than 36 hours and less than 48 hours" is, for example, 37, 38, 39, 40, 41, 42, 43, 44, 45, 46, or 47 hours. These values (N hours) can be arbitrarily selected as the lower limit (N hours or more, or exceeding N hours) and/or the upper limit (N hours or less, or less than N hours) when determining a new activation duration range for performing the present invention that is included in "more than 36 hours and less than 48 hours." The activation duration in a preferable embodiment of the present invention is 37 to 44 hours, more preferably 39 to 44 hours, even more preferably 39 to 42 hours, and particularly preferably 39 to 41 hours.

The activation step can be ended by replacing the medium containing a CD3 agonist and/or a CD28 agonist with a medium free of a CD3 agonist and a CD28 agonist, or by diluting the medium containing a CD3 agonist and/or a CD28 agonist. If the activation step is ended by diluting the medium, the activation duration in the present invention does not include the time required for culturing T cells in such a diluted medium. For example, if the activation step using TransACT is ended by diluting the medium, the activation step can be ended by diluting the medium to 70% or less (preferably 50% or less).

In a preferable embodiment of the present invention, the culture period for performing the activation method of the present invention is a culture period that can attain high CAR expression efficiency, as compared to the CAR expression efficiency of the control group in which culturing is conducted under the same conditions as the present invention except that the culture period is changed to 24, 48, or 72 hours. For the efficiency of CAR expression, (a) the percentage of CAR-expressing cells in a cell population or (b) the number of CAR-expressing cells in a cell population can be used as an index. If at least one of these indices (a) and (b) is higher than that of the control group at the stage after step 3 (the culture step of the method for producing genetically modified T cells; production method of the present invention) described below, it is evaluated that the CAR expression efficiency is increased.

In the activation method (activation step) of the present invention, the culture time for activating T cells is set to a specific range; however, other embodiments of culture, such as the culture method, medium, and other culture conditions (temperature, atmosphere, cell density, etc.) are in accordance with known or common T-cell activation methods, or may be suitably adjusted to suit the present invention based on the known or common T-cell activation methods. The cell density at the time of seeding in the activation step is, for example, 1.0×10⁵ to 1.0×10⁷ cells/mL, and preferably 2.0×10⁵ to 6.0×10⁶ cells/mL.

The culture container in the activation step is not particularly limited, and can be suitably selected from plates, dishes, Petri dishes, flasks, bags, bottles, tanks (culture tanks), and the like according to the scale of culture. For example, a closed automated culture system, such as CliniMACS Prodigy (trademark) (Miltenyi Biotec) can be used.

In the activation step, a known or common medium used for culturing T cells can be used, and a necessary component can be appropriately supplemented to a medium.

Examples of the medium in the activation step include AIM V, X-VIVO-15, NeuroBasal, EGM2, TeSR, BME, BGJb, CMRL1066, Glasgow's MEM, Improved MEM Zinc Option, IMDM, 199 medium, Eagle's MEM, αMEM, DMEM, Ham, RPMI-1640, Fischer's medium, and other commercially available products for culturing T cells (such as CTS^{™} OpTmizer^{™} T-Cell Expansion Basal Medium (Thermo Fisher Scientific), CTS^{™} OpTmizer^{™} Pro Serum Free Medium (Thermo Fisher Scientific), CTS^{™} OpTmizer^{™} Pro (Thermo Fisher Scientific), and CTS^{™} OpTmizer^{™} T-Cell Expansion Supplement (Thermo Fisher Scientific)). These media may be used singly or as a mixture of two or more.

The medium may be a serum-containing or serum-free medium or a Xenofree medium. From the viewpoint of preventing contamination by components derived from different types of animals, the serum may be derived from the same animal as that of the cells to be cultured. The serum-free medium refers to an unprocessed or unpurified serum-free medium, and can therefore include a medium containing a purified blood-derived component or an animal tissue-derived component (e.g., growth factor). The medium may or may not contain any substitute for serum. Examples of the serum substitute may include materials appropriately containing albumin (albumin substitutes such as lipid-rich albumin, bovine albumin, recombinant albumin, and humanized albumin; plant starch; dextran; and protein hydrolysates), transferrin (or other iron transporters), fatty acids, insulin, collagen precursors, trace elements, 2-mercaptoethanol, 3'-thioglycerol (α-monothioglycerol, MTG), or equivalents. Commercially available materials, such as knockout serum replacement (KSR), chemically defined lipid concentrated (Thermo Fisher Scientific), and GlutaMAX (Thermo Fisher Scientific) can be used. The medium may be a serum-free medium (SFM). For example, the medium may contain a B-27 (trademark) supplement, a Xenofree B-27 (trademark) supplement, an NS21 supplement, a GS21 (trademark) supplement, or combinations thereof, at a concentration effective for producing T cells from a 3D cell aggregate.

The medium may contain one or more members selected from the group consisting of biotin; DL alpha-tocopherol acetate; DL alpha-tocopherol; vitamins such as vitamin A (acetate); bovine serum albumin (BSA) or human albumin, fatty acid-free fraction V; catalase; human recombinant insulin; human transferrin; proteins such as superoxide dismutase; corticosterone; D-galactose; ethanolamine HCl; glutathione (reduced form); L-carnitine HCl; linoleic acid; linolenic acid; progesterone; putrescine 2HCl; sodium selenite; and T3 (triiodo-L-thyronine); and PSG (penicillin, streptomycin, and L-glutamine). The medium may also include externally added ascorbic acid or the derivative thereof (e.g., ascorbic acid 2-phosphate: PAA) . The medium may contain one or more members selected from the group consisting of fatty acids or lipids, amino acids (such as non-essential amino acids), vitamins, growth factors, cytokines, antibiotics, antioxidants, 2-mercaptoethanol, pyruvic acids, buffers, and inorganic salts, each of which added externally.

The medium may also contain an externally added cytokine. Examples of the cytokine include FLT3 ligand (FLT3L), interleukin-7 (IL-7), stem cell factor (SCF), thrombopoietin (TPO), IL-2, IL-3, IL-4, IL-6, IL-12, IL-15, IL-18, IL-21, TNF-alpha, TGF-beta, interferon-gamma, interferon-lambda, TSLP, thymopentin, pleotrophin, and midkine. These cytokines may be used alone or in a combination of two or more. A preferred cytokine is IL-2.

In one preferable embodiment of the present invention, the CD3 agonist and/or the CD28 agonist is supported on a matrix of mobile polymer chains. The product MACS (trademark) GMP T-Cell TransACT^{™} (Miltenyi Biotec) used in the Examples is a product in which a CD3 agonist and a CD28 agonist are supported on a matrix of mobile polymer chains, and corresponds to this embodiment.

The definition and embodiment of the "matrix of mobile polymer chains" (in this specification sometimes referred to as the "mobile matrix") in the present invention is the same as the definition and embodiment of the term described in Patent Literature 1 (WO2014/048920), and the description in Patent Literature 1 can be used as a reference in the present invention (this specification). The "mobile matrix" may be made of collagen, purified proteins, purified peptides, polysaccharides, glycosaminoglycans, or extracellular matrix compositions. Examples of the polysaccharide include cellulose ethers, starch, gum arabic, agarose, dextran, chitosan, hyaluronic acid, pectin, xanthan, guar gum, or alginate. Other polymers include polyesters, polyethers, polyacrylates, polyacrylamides, polyamines, polyethyleneimines, polyquaternium polymers, polyphosphazenes, polyvinyl alcohols, polyvinyl acetates, polyvinylpyrrolidones, block copolymers, and polyurethanes. The mobile matrix is preferably a polymer of dextran.

In one preferable embodiment of the present invention, the CD3 agonist and/or the CD28 agonist is supported on a bead. Examples include magnetic beads such as Dynabeads Human T-Activator CD3/CD28 (Thermo Fisher Scientific).

The CD3 agonist and/or CD28 agonist is "supported on," i.e., "attached to," such a mobile matrix or bead. A substance (CD3 agonist and/or CD28 agonist) can be attached or coupled to the mobile matrix by a variety of methods known and available in the art. The attachment may be covalent or noncovalent, electrostatic, or hydrophobic, and may be accomplished by a variety of attachment means, including chemical, mechanical, enzymatic, or other means whereby the substance is capable of stimulating the cells. For example, the antibody may be directly attached to the matrix or bead, or may be indirectly attached to the matrix or bead via an anti-isotype antibody. As another example, the antibody may be attached using protein A or protein G, or other non-specific antibody binding molecules attached to matrices or beads. Alternatively, the substance may be attached to the matrix or bead by chemical means, such as cross-linking to the matrix or bead.

In one embodiment of the present invention, the CD3 agonist and/or CD28 agonist may be a DNA-based T-cell activator in which CD3 and CD28 antibodies are conjugated via complementary oligonucleotides to a single-stranded DNA scaffold (Keskar et al., J Immunother, 2020, vol. 43, No. 8, 231-235).

### Method for Producing Genetically Modified T Cells

The production method of the present invention is a method for producing genetically modified T cells, comprising the following steps 1 to 3:
Step 1: Activating T cells by the activation method of the present invention.
Step 2: Introducing an exogenous gene into the activated T cells (referred to as the "transduction step" in this specification).
Step 3:Culturing the T cells into which the exogenous gene has been introduced (referred to as the "culture step" in this specification).

### Step 1: Activation Step

The activation step is a step of activating T cells by carrying out the activation method of the present invention. The aforementioned matter describing the activation method of the present invention explains the details of the activation step.

### Step 2: Transduction Step

The transduction step is a step of introducing an exogenous gene into the T cells obtained by the activation step.

The "exogenous gene" is a gene to be introduced from the outside in order to express the desired protein in the T cells, and can be suitably selected depending on the use of T cells to be produced. One or more exogenous genes can be used.

In one embodiment of the present invention, the exogenous gene can include a gene for expressing a CAR. In one embodiment of the present invention, the exogenous gene can include a gene for expressing a CAR, and a gene for expressing a cytokine and/or a chemokine. For example, by expressing a CAR and a specific combination of a cytokine and a chemokine (preferably IL-7 and CCL19) using exogenous genes, the anti-tumor activity of T cells can be enhanced. As other exogenous genes, an antigen-specific TCR (T-cell receptor) and STAR receptor (synthetic TCR and antigen receptor) (Liu et al., Sci. Transl. Med. 2021, vol. 13, issue 586) can be introduced into T cells, for example.

### (1) CAR

As with general or known CARs, the CARs expressed by the T cells are basically configured such that peptides at sites of (i) a target recognition site that recognizes cell-surface antigens of cancer cells (e.g., single-chain antibody), (ii) a transmembrane region, and (iii) a signal transduction region that induces the activation of T cells are linked via a spacer, as needed.

(i) The target recognition site can be an antibody (e.g., single-chain antibody) that recognizes a cell-surface antigen of cancer cells. The single-chain antibody (i) that recognizes a cell-surface antigen of cancer cells is typically a single-chain variable fragment (scFv) composed of a light-chain variable region and a heavy-chain variable region derived from antigen-binding sites of a monoclonal antibody that specifically binds to the antigen, and a linker peptide that links such regions. (i) The target recognition site may recognize any target as long as the CAR can specifically exhibit anti-tumor effects against cancer cells. For example, it may be an antibody (single-chain antibody) that recognizes the constant region of the antibody that specifically binds to the cell surface antigen of cancer cells.

The "cell-surface antigen of cancer cells" targeted by the CAR may be a biomolecule that is specifically expressed in cancer cells and their progenitor cells, a biomolecule that has been newly expressed by canceration of cells, or a biomolecule with an increased expression level in cancer cells as compared with normal cells. Such an antigen is generally referred to as "tumor-associated antigen" (TAA), and examples include BCMA, B7-H3, B7-H6, CD7, CD10, CD19, CD20, CD22, CD23, CD24, CD30, CD33, CD34, CD38, CD41, CD44, CD56, CD70, CD74, CD97, CD123, CD133, CD138, CD171, CD248, CAIX, CEA, c-Met, CS1 (CD319), CSPG4, CLDN6, CLD18A2, CYP1B1, DNAM-1, GD2, GD3, GM2, GFRα4, GPC3, GPR20, GPRC5D, globoH, Gp100, GPR20, GPRC5D, EGFR, EGFR variant, EpCAM, EGP2, EGP40, FAP, FITC, HER2, HER3, HPV E6, HPV E7, hTERT, IgG κ chain, IL-11Ra, IL-13Ra2, KIT, Lewis A, Lewis Y, Legumain, LMP1, LMP2, Ly6k, LICAM, MAD-CT-1, MAD-CT-2, MAGE-A1, Melanoma-associated antigen 1, MUC1, MUC16, NA-17, NY-BR-1, NY-ESO-1, O-acetyl-GD2, h5T4, PANX3, PDGRFb, PLAC1, polysialic acid, PSCA, PSMA, RAGE1, ROR1, sLe, SSEA-4, TARP, TAG-72, TEM7R, Tn antigen, TRAIL receptor, TRP2, TSHR, α fetoprotein, mesothelin, folic acid receptor α (FRα), folic acid receptor β (FRβ), FBP, UPK2, VEGF-R2, and WT-1, but is not limited to these examples.

The transmembrane region (ii) is a polypeptide serving as a region for fixing the CAR to the cell membrane of T cells. Examples of the transmembrane region include those derived from BTLA, CD3ε, CD4, CD5, CD8, CD9, CD16, CD22, CD28, CD33, CD37, CD45, CD64, CD80, CD86, CD134, CD137, CD154, 4-1BB (CD137), CTLA-4, GITR, ICOS, LAG3, OX40, SLAMF4 (CD244, 2B4), or the α or β chain of a T-cell receptor. Alternatively, mutant transmembrane regions having an amino acid sequence with an identity of 80% or more, 85% or more, 90% or more, 910 or more, 92% or more, 93% or more, 94% or more, 95% or more, 96% or more, 97% or more, 98% or more, or 99% or more, to the natural amino acid sequence of the aforementioned transmembrane regions can also be used. In one embodiment of the present invention, the transmembrane region of CD8 is preferable as such a transmembrane region.

To the transmembrane region, a hinge region that is a peptide (oligopeptide or polypeptide) consisting of any amino acid sequence having a length of 1 to 100 amino acids and preferably 10 to 70 amino acids, may be linked. Examples of the hinge region include hinge regions derived from CD3, CD8, KIR2DS2, or IgG4, IgD, or other immunoglobulins. Alternatively, mutant hinge regions having an amino acid sequence with an identity of 80% or more, 85% or more, 90% or more, 910 or more, 92% or more, 93% or more, 94% or more, 95% or more, 96% or more, 97% or more, 98% or more, or 99% or more, to the natural amino acid sequences of the aforementioned hinge regions can also be used. In one embodiment of the present invention, the hinge region is preferably the hinge region of CD8.

The signal transduction region (iii) that induces T-cell activation is a polypeptide serving as a region for transmitting signals within T cells when the target recognition site (e.g., single-chain antibody) recognizes the target (e.g., cell-surface antigen of cancer cells) and binds thereto. Examples of the signal transduction region include one or more intracellular regions selected from the group consisting of MHC class I molecules, TNF receptor proteins, immunoglobulin-like proteins, cytokine receptors, integrins, activated NK cell receptors, Toll-like receptors, B7-H3, SAFER, BTLA, BY55 (CD160), CD2, CD3ζ, CD4, CD7, CD8α, CD8β, CD11a, CD11b, CD11c, CD11d, CD18, CD19, CD19a, CD27, CD28, CD29, CD30, CD40, CD49a, CD49D, CD49f, CD69, CD84, CD96 (Tactile), CD103, 4-1BB (CD137), CDS, CEACAM1, CRTAM, CNAM1 (CD226), DAP10, Fc Receptor-associated γ chain, GADS, GITR, HVEM (LIGHTR), IA4, ICAM-1, ICOS (CD278), IL2Rβ, IL2Ry, IL7Rα, ITGA4, ITGA6, ITGAD, ITGAE, ITGAL, ITGAM, ITGAX, ITGB1, ITGB2, ITGB7, KIRDS2, Ly9 (CD229), LAT, LFA-1 (CD11a/CD18), LIGHT, LTBR, NKG2C, NKG2D, NKp30, NKp44, NKp46, NKp80 (KLRF1), OX40, PAG/Cbp, PSGL1, SELPLG (CD162), SEMA4D (CD100), SLAM (SLAMF1, CD150, IPO-3), SLAMF4 (CD244, 2B4), SLAMF6 (NTB-A, Ly108), SLAMF7, SLAMF8 (BLAME), SLP-76, TNFR2, TRANCE/RANKL, VLA1, and VLA-6. Alternatively, mutant signal transduction regions (intracellular regions) having an amino acid sequence with an identity of 80% or more, 85% or more, 90% or more, 910 or more, 92% or more, 93% or more, 94% or more, 95% or more, 96% or more, 97% or more, 98% or more, or 99% or more, to the natural amino acid sequence of the aforementioned signal transduction region (intracellular region) can also be used. In one embodiment of the present invention, the signal transduction region preferably contains the intracellular regions of CD28 and CD3ζ, intracellular regions of 4-1BB and CD3ζ, or intracellular regions of CD28, 4-1BB, and CD3ζ.

When the signal transduction region contains a plurality of intracellular regions, the intracellular regions may be linked to each other via linker peptides (oligopeptides or polypeptides) consisting of 2 to 10 amino acids. Examples of such linker peptides include a peptide consisting of a glycine-serine contiguous sequence.

Both between the antigen-recognition site (e.g., single-chain antibody) and the transmembrane region, and between the transmembrane region and the signal transduction region, a spacer region that is a peptide (oligopeptide or polypeptide) consisting of any amino acid sequence having a length of 1 to 100 amino acids, and preferably 10 to 50 amino acids, may be included. Examples of the spacer region include a peptide consisting of a glycine-serine contiguous sequence.

The amino acid sequence of the aforementioned CAR expressed by T cells may be appropriately set depending on the application of T cells, typically, their functions as an active component of a medicament for treating cancer. As the amino acid sequence of the single-chain antibody against the cell-surface antigen of cancer cells, which is an example of the target recognition site (i), various amino acid sequences are known, and information on such amino acid sequences can also be used in the present invention. Alternatively, a new antibody against a desired cell-surface antigen of cancer cells may be produced according to a conventional method, and information obtained by determining the amino acid sequence of the new antibody (preferably heavy-chain and light-chain variable regions, especially CDRs) may be used in the present invention. Further, various amino acid sequences derived from humans and mammals other than humans are known as the amino acid sequences of the transmembrane region (ii) and the T-cell activation signal transduction region (iii), and such amino acid sequences are also registered in databases such as NCBI (National Center for Biotechnology Information; http://www.ncbi.nlm.nih.gov/guide/), and UniProt (The Universal Protein Resource; https://www.uniprot.org). Therefore, such information can also be used in the present invention. Even when the target recognition site (i) is an antibody that recognizes the constant region of the antibody that binds to the surface antigen of cancer cells, it can be obtained or produced according to a conventional method.

### (2) Cytokine

Various cytokines are known as cytokines that can be expressed by T cells together with a CAR. In the present invention as well, known cytokines can be expressed by exogenous genes. Examples of the cytokine include IL-7, IL-15, IL-18, IL-21, and IL-27. One or more cytokines may be expressed by T cells. The cytokine is preferably derived from the same animal species (human or a non-human mammal) as that of the T cells into which the exogenous gene is introduced (receptor for a cytokine on the T cells). The amino acid sequences of various natural cytokines derived from human or non-human mammals are known and registered in databases such as NCBI (National Center for Biotechnology Information; http://www.ncbi.nlm.nih.gov/guide/) and UniProt (The Universal Protein Resource; https://www.uniprot.org). Therefore, such information can also be used in the present invention.

The term "cytokine" in the present invention includes not only natural full-length proteins, but also proteins (polypeptides) that have been modified in various ways to retain or enhance their functions as cytokines. Specifically the cytokine in the present invention is (a) a whole protein (polypeptide) consisting of the natural amino acid sequence, or a part thereof (functional partial polypeptide), (b) a variant in which one or a plurality of amino acids are deleted, replaced, or added to the natural amino acid sequence, or (c) a fusion protein in which a full-length protein or a partial polypeptide is linked at the N- or C-terminus to another protein (e.g., a signal peptide and a subunit of a receptor protein). Examples of the variant (b) include those having an amino acid sequence having an identity of 80% or more, 85% or more, 90% or more, 910 or more, 92% or more, 93% or more, 94% or more, 95% or more, 96% or more, 97% or more, 98% or more, or 99% or more, as a whole or a partial region (domain), with the amino acid sequences of natural cytokines.

### (3) Chemokine

Various chemokines are known as chemokines that can be expressed by T cells together with a CAR. In the present invention as well, a known chemokine can be expressed by an exogenous gene. Examples of the chemokine include CCL19. One or more chemokines may be expressed by T cells. The chemokine is preferably derived from the same animal species (human or non-human mammals) as that of the T cells into which an exogenous gene is introduced (receptor for a chemokine on the T cells). The amino acid sequences of various natural cytokines derived from human and non-human mammals are known and registered in databases such as NCBI (National Center for Biotechnology Information; http://www.ncbi.nlm.nih.gov/guide/) and UniProt (The Universal Protein Resource; https://www.uniprot.org). Therefore, such information can also be used in the present invention.

The term "chemokine" in the present invention encompasses not only natural full-length proteins, but also proteins (polypeptides) that have been modified in various ways to retain or enhance their functions as chemokines. Specifically, the "chemokine" in the present invention indicates (a) a whole protein (polypeptide) consisting of the natural amino acid sequence, and a part thereof (functional partial polypeptide) or (b) a variant in which one or a plurality of amino acids are deleted, replaced, or added to the natural amino acid sequence. Examples of the variant (b) include those having an amino acid sequence having an identity of 80% or more, 85% or more, 90% or more, 910 or more, 92% or more, 93% or more, 94% or more, 95% or more, 96% or more, 97% or more, 98% or more, or 99% or more, as a whole or a partial region (domain), with the amino acid sequences of natural chemokines.

The means for introducing the exogenous gene into the T cells is not particularly limited, and various known or general means can be used. Typically, the exogenous gene is introduced into the T cells using an expression vector, and is expressed. The expression vector may be linear or cyclic, and may be a non-viral vector such as a plasmid, a viral vector, or a transposon vector.

The means for introducing the expression vector into the T cells can be made appropriate according to the embodiment. For example, the expression vector can be introduced into the T cells by a known method, such as a virus infection method, a calcium phosphate method, a lipofection method, a microinjection method, or an electroporation method. The expression vector can be prepared in a form suitable for use in each method by known means, or using commercially available kits as appropriate (according to the instructions). In the transduction step, such a preparation is brought into contact with T cells; generally, T cells may be cultured in a medium to which a preparation containing the expression vector is added.

In one embodiment of the present invention, the expression vector is introduced into the T cells by a virus infection method. Examples of viral vectors include retroviral vectors, lentiviral vectors, adenoviral vectors, and adeno-associated viral vectors. Preferable examples of retroviral vectors include pMSGV vectors (Tamada K et al., ClinCancer Res 18:6436-6445(2002)), pMSCV vectors (produced by Takara Bio Inc.), and SFG vectors. When a retroviral vector is used, a gene contained in the vector is incorporated into the genome of the host cells (T cells in this invention), which allows the gene to be stably expressed for a long period of time. When these viral vectors are used, a vector containing the desired exogenous gene and a packaging vector (plasmid) of each virus may be transfected into host cells using a corresponding commercially available kit to produce a recombinant virus, and then the T cells may be infected with the obtained recombinant virus. Examples of the commercially available kit for viral vectors include Retrovirus Packaging Kit Eco (produced by Takara Bio Inc.). Examples of the host cell include GP2-293 cells (produced by Takara Bio Inc.), Plat-GP cells (produced by Cosmo Bio Co., Ltd.), PG13 cells (ATCC CRL-10686), and PA317 cells (ATCC CRL-9078).

When a plurality of exogenous genes are used, all of the exogenous genes may be contained in one expression vector, all of the exogenous genes may be separately contained in different expression vectors, or part of the plurality of exogenous genes may be contained in one expression vector, and the other genes may be separately contained in different expression vectors. When one expression vector contains a plurality of exogenous genes, the order in which those exogenous genes are arranged from the upstream side to the downstream side is not particularly limited.

The exogenous gene can be composed of a nucleic acid (polynucleotide) having a base sequence encoding the amino acid sequence of the desired protein or polypeptide to be expressed by T cells, such as CARs, cytokines, and chemokines as described above. Those skilled in the art would be able to design and produce an expression vector capable of expressing a desired protein (polypeptide) in the T cells. The nucleic acids contained in the expression vector may be produced by chemically synthesizing DNA or may be produced (cloned) as cDNA.

The expression vector may contain (in the case of the aforementioned combinations of a plurality of expression vectors, the expression vectors each independently may contain) the sequence of a promoter, terminator, enhancer, start codon, stop codon, polyadenylation signal, nuclear localization signal (NLS), multicloning site (MCS), or the like, that is involved in the expression of each exogenous gene, as required, in addition to the base sequence (exogenous gene) encoding the desired protein or polypeptide. When one expression vector contains a plurality of exogenous genes, a gene encoding a self-cleaving peptide (e.g., 2A peptide) or IRES (internal ribozyme entry site) may be inserted between the exogenous genes. The expression vector may further contain a nucleic acid (base sequence) encoding "a functional gene" such as a reporter gene (typically, a gene encoding a fluorescent protein of each color), a drug selection gene (such as a kanamycin resistance gene, an ampicillin resistance gene, and a puromycin resistance gene), and a suicide gene (such as a gene encoding diphtheria toxin A, herpes simplex thymidine kinase (HSV-TK), carboxypeptidase G2 (CPG2), carboxyl esterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella-zoster virus thymidine kinase (VZV-TK), xanthine-guanine phosphoribosyl transferase (XGPRT), or inducible caspase 9).

The "nucleic acid" may be any molecule obtained by polymerizing a nucleotide and a molecule having the same function as the nucleotide. Examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog. The nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid. Specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

The culture vessel, medium, added components, and other culture conditions in the transduction step can be the same as those described with respect to the activation step in the activation method of the present invention, and can be selected appropriately from among those listed. However, the culture vessel, medium, and added components in the transduction step are not necessarily the same as those in the activation step, and can be appropriately changed.

### Step 3: Culture Step

The culture step is a step of culturing the T cells into which the exogenous gene has been introduced. The culture period in the culture step is not particularly limited as long as it is long enough for the desired protein to be expressed by T cells. The culture period is, for example, 20 days, and preferably 3 to 7 days.

If the expression vector contains a drug resistance gene as a functional gene, in order to select T cells into which the expression vector has been introduced, a drug corresponding to the used drug resistance gene may be added to the medium in the culture step, thus culturing the T cells. If the expression vector contains a gene (reporter gene) encoding a fluorescent protein as a functional gene, T cells into which an expression vector has been introduced may be observed with fluorescence microscopy in or after the culture step, or T cells into which the expression vector have been introduced may be sorted with a cell sorter.

The culture vessel, medium, added components, and other culture conditions in the culture step can be the same as those described with respect to the activation step in the activation method of the present invention, and can be appropriately selected from those listed. However, the culture vessel, medium, and added components in the culture step are not necessarily the same as those in the activation step, and can be appropriately changed.

The application of the cell population containing genetically modified T cells obtained by the production method of the present invention is not particularly limited. The cell population can be used for any desired purpose. For example, the cell population can be used to produce a medicament (cell preparation).

The medicament (cell preparation) contains genetically modified T cells, and may further contain other components, as required. Those skilled in the art would be able to appropriately prepare such a medicament using genetically modified T cells in consideration of the application (disease to be treated, subject to be administered, or the like) and dosage form.

The medicament can be a medicament (anticancer agent) to treat cancer corresponding to the cell-surface antigen of cancer cells (cancer-specific antigen) targeted by the CAR expressed by genetically modified T cells. Accordingly, the type of cancer targeted by the medicament is not particularly limited as long as cancer cells expressing the antigen targeted by the CAR are contained in the cancer tissue, and a certain level of therapeutic effect is observed by the CAR-T cells. Examples of the cancer that can be targeted by the medicament include cancers such as adenocarcinoma, squamous cell carcinoma, adenosquamous carcinoma, undifferentiated cancer, large-cell cancer, small-cell cancer, skin cancer (e.g., melanoma and Merkel cell cancer), breast cancer, prostate cancer, bladder cancer, vaginal cancer, neck cancer, head and neck cancer, uterine cancer, cervical cancer, liver cancer, kidney cancer, pancreatic cancer, spleen cancer, lung cancer, non-small-cell lung cancer, tracheal cancer, bronchial cancer, colon cancer, rectal cancer, small-intestine cancer, colorectal cancer, stomach cancer, esophageal cancer, gallbladder cancer, testicular cancer, ovarian cancer, fallopian tube cancer, and nasopharyngeal cancer; cancers of bone tissue, cartilage tissue, adipose tissue, muscle tissue, vascular tissue, and hematopoietic tissue; sarcomas, such as chondrosarcoma, Ewing's sarcoma, rhabdomyosarcoma, malignant hemangioendothelioma, osteosarcoma, and soft tissue sarcoma; blastomas, such as hepatoblastoma, medulloblastoma, nephroblastoma, neuroblastoma, pancreatoblastoma, pleuropulmonary blastoma, and retinoblastoma; embryonic cell tumors; lymphoma; leukemia, acute myeloid leukemia, and multiple myeloma. Preferably, examples include cancers sensitive to NK cells (such as melanoma, Merkel cell cancer, colorectal cancer, kidney cancer, breast cancer, ovarian cancer, fallopian tube cancer, cervical cancer, liver cancer, lung cancer, non-small-cell lung cancer, head and neck cancer, small-intestine cancer, prostate cancer, bladder cancer, rectal cancer, pancreatic cancer, Ewing's sarcoma, rhabdomyosarcoma, nasopharyngeal cancer, esophageal cancer, biliary tract cancer, neuroblastoma, osteosarcoma, acute myeloid leukemia, multiple myeloma, lymphoma, and leukemia). More preferably, examples include melanoma, colorectal cancer, kidney cancer, multiple myeloma, lymphoma, and leukemia.

Examples of components that can be contained in the medicament other than the genetically modified T cells include pharmaceutically acceptable additives, more specifically, saline, buffered saline, cell culture media, dextrose, water for injection, glycerol, ethanol, stabilizers, solubilizers, surfactants, buffers, preservatives, isotonic agents, fillers, and lubricants.

The medicament can be used by being administered to a subject in need of cancer treatment (such as cancer patients and cancer-bearing animals) by the same method as for known genetically modified T cells (such as CAR-T cells). Examples of the administration method include intratumor, intravenous, intraarterial, intramuscular, subcutaneous, and intraperitoneal injections.

The amount of the genetically modified T cells contained in the medicament can be appropriately adjusted, for example, depending on the application, dosage form, intended therapeutic effect, and the like, in consideration of the type, location, and severity of cancer, the age, body weight, and condition of the subject to be treated, and the like. For example, the medicament can be formulated so that the CAR-T cells are administered in an amount of normally 1×10⁴ to 1×10¹⁰, preferably 1×10⁵ to 1×10⁹, more preferably 5×10⁶ to 5×10⁸, in a single dose.

The administration interval of the medicament is not particularly limited and can be appropriately adjusted in consideration of the amount of the T cells of the present invention to be administered at one time. The medicament can be independently administered, for example, 4 times, 3 times, twice, or once a day, every other day, every 3 days, every 4 days, every 5 days, every 6 days, once a week, every 8 days, every 9 days, every 10 days, twice a week, once a month, or twice a month.

When the medicament is used for cancer treatment, it can be used in combination with a known anticancer agent. Examples of the anticancer agent include alkylating drugs, such as cyclophosphamide, bendamustine, ifosfamide, and dacarbazine; antimetabolites, such as pentostatin, fludarabine, cladribine, methotrexate, 5-fluorouracil, 6-mercaptopurine, and enocitabin; molecular targeted drugs, such as rituximab, cetuximab, and trastuzumab; kinase inhibitors, such as imatinib, gefitinib, erlotinib, afatinib, dasatinib, sunitinib, and trametinib; proteasome inhibitors, such as bortezomib; calcineurin inhibitors, such as cyclosporine and tacrolimus; antitumor antibiotics, such as idarubicin, doxorubicin, and mitomycin C; plant alkaloids, such as irinotecan and etoposide; platinum preparations, such as cisplatin, oxaliplatin, and carboplatin; hormone therapeutic drugs, such as tamoxifen and bicalutamide; and immunoregulatory drugs, such as interferon, nivolumab, and pembrolizumab. When the target recognition site of CAR is an antibody that recognizes the constant region of the antibody that specifically binds to a cell surface antigen of cancer cells, the genetically modified T cells can be used as a medicament for treating a cancer that corresponds to the cell surface antigen. In this case, a medicament containing genetically modified T cells can be used together with the medicament containing an antibody that specifically binds to the cell surface antigen.

### Definition

"Induced pluripotent stem cells (iPSc)" refer to cells obtained by reprogramming mammalian somatic cells or undifferentiated stem cells by introduction with specific factors (nuclear reprogramming factors). Currently, there are many different types of induced pluripotent stem cells. Usable examples include iPSc established by Yamanaka et al. by introducing four factors, Oct3/4, Sox2, Klf4, and c-Myc, into mouse fibroblasts (Takahashi K., Yamanaka S., Cell, (2006) 126: 663-676), as well as human cell-derived iPSc established by introducing the same four factors into human fibroblasts (Takahashi K., Yamanaka S., et al. Cell, (2007) 131: 861-872), Nanog-iPS cells established by introducing the above four factors, followed by screening using the expression of Nanog as an index (Okita K., Ichisaka T., and Yamanaka S. (2007). Nature 448, 313-317), iPSc produced by a method that does not include c-Myc (Nakagawa M., Yamanaka S., et al. Nature Biotechnology, (2008) 26, 101-106), and iPSc established by introducing six factors by a virus-free method (Okita K. et al. Nat. Methods 2011 May; 8(5): 409-12, Okita K. et al. Stem Cells. 31(3): 458-66). Other usable examples include induced pluripotent stem cells produced by Thomson et al. and established by introducing four factors, OCT3/4, SOX2, NANOG, and LIN28 (Yu J., Thomson J.A., et al., Science (2007) 318: 1917-1920), induced pluripotent stem cells produced by Daley et al. (Park I.H., Daley G.Q., et al., Nature (2007) 451: 141-146), induced pluripotent stem cells produced by Sakurada et al. (JP2008-307007A), and the like.

Other usable examples are any of the induced pluripotent stem cells known in the art and disclosed in all of the published articles (e.g., Shi Y., Ding S., et al., Cell Stem Cell, (2008) Vol. 3, Issue 5, 568-574; Kim J.B., Scholer H.R., et al., Nature, (2008) 454, 646-650; Huangfu D., Melton D.A., et al., Nature Biotechnology, (2008) 26, No. 7, 795-797), or patents (e.g., JP2008-307007A, JP2008-283972A, US2008/2336610, US2009/047263, WO2007/069666, WO2008/118220, WO2008/124133, WO2008/151058, WO2009/006930, WO2009/006997, and WO2009/007852).

As "induced pluripotent stem cells," various iPSc strains established by NIH, RIKEN, Kyoto University, etc. can be used. Examples of human iPSc strains include HiPS-RIKEN-1A strain, HiPS-RIKEN-2A strain, HiPS-RIKEN-12A strain, and Nips-B2 strain (RIKEN); 253G1 strain, 201B7 strain, 409B2 strain, 454E2 strain, 606A1 strain, 610B1 strain, and 648A1 strain (Kyoto University); and the like. Alternatively, clinical-grade cell lines provided by Kyoto University, Cellular Dynamics International, or the like, and cell lines for research and clinical use produced using such cell lines may be used.

Regarding "embryonic stem cells (ESC)," various mouse ESC strains established by Ingenious Targeting Laboratory, RIKEN, etc. can be used as mouse ESC, and various human ESC strains established by NIH, RIKEN, Kyoto University, Cellartis, etc. can be used as human ESC. Usable examples of human ESC strains include CHB-1 to CHB-12 strains, RUES1 strain, RUES2 strain, HUES1 to HUES28 strains, etc. of NIH; H1 strain, H9 strain, etc. of WisCell Research; and KhES-1 strain, KhES-2 strain, KhES-3 strain, KhES-4 strain, KhES-5 strain, SSES1 strain, SSES2 strain, SSES3 strain, etc. of RIKEN. Alternatively, clinical-grade cell lines and cell lines for research and clinical use produced using such cell lines may be used.

The term "comprise(s)" or "comprising" means that although elements following these terms are included, the inclusion is not limited to the elements. Therefore, these terms suggest inclusion of elements following them, but do not suggest exclusion of any other elements. The term "consist(s) of" or "consisting of" means that any elements following these terms are included, and that the inclusion is limited to the elements. Therefore, the term "consist(s) of" or "consisting of" indicates that the listed elements are required or essential, and that there are substantially no other elements. The term "consist(s) essentially of" or "consisting essentially of" means that any elements following these terms are included, and that there is a limitation to other elements that do not affect the activity or action specified in the present disclosure for the above elements. Therefore, the term "consist(s) essentially of" or "consisting essentially of" indicates that the listed elements are required or essential, while other elements are optional, and may be or may not be present depending on whether they affect the activity or action of the listed elements.

As to the cells, compounds, and other substances in this specification, those described in the singular form and those described in the plural form are mutually exchangeable unless otherwise specified.

### Examples

### (I) Material and Method

### (1) Medium

A 2.6% CTS^{™} OpTmizer^{™} T-Cell Expansion Supplement (Thermo Fisher Scientific), 1% L-Glutamine (Thermo Fisher Scientific), 1% Streptomycin, and 2% CTS^{™} Immune Cell SR (Thermo Fisher Scientific) were added to a CTS^{™} OpTmizer^{™} T-Cell Expansion Basal Medium (Thermo Fisher Scientific) to produce a basic medium. MACS GMP IL-2 (Miltenyi Biotec) was added in an amount of 20 IU/mL or 40 IU/mL to the basic medium to form a culture medium.

### (2) Production of CAR-T Cells on Small Scale Using PBMC

Human PBMC (4 donors: Donors A, B, C, and D) were thawed and then diluted in the culture medium to a concentration of 1.0×10⁶ cells/mL. The cells were seeded on a culture plate so that the ratio of the cell suspension to MACS GMP T-Cell TransACT (Miltenyi Biotec) was 17.5:1, and cultured for 24 to 72 hours (activation step). After the activated cells were centrifuged, and the medium was replaced with a fresh culture medium, the cells were seeded at a concentration of 5.0×10⁴ cells/cm² on a culture plate that was pre-coated with Retronectin (Takara Bio Inc.) and Retrovirus beforehand, and cultured until the next day (gene transduction step). In the gene transduction step, an exogenous gene that comprises a CAR gene (comprising anti-GPC3 scFv, CD8 hinge region, CD8 transmembrane region, CD28 intracellular region, 4-1BB intracellular region, and CD3ζ intracellular region), IL-7 gene, and CCL19 gene, wherein those genes were each connected through a nucleotide sequence encoding self-cleaving peptide (2A peptide), was introduced. The cells were washed with the culture medium, and then all of the cells were cultured in the culture medium in the culture plate for 4 to 6 days.

### (3) Production of CAR-T Cells on Clinical Scale Using T Cells

After Leukopak (human leukocyte apheresis product, HemaCare) (3 donors: Donors E, F, and G) was thawed, T cells were isolated using the CliniMACS CD4 GMP Microbeads (Miltenyi Biotec) and the CliniMACS CD8 GMP Microbeads (Miltenyi Biotec) in the CliniMACS Prodigy (Miltenyi Biotec). Isolated T cells were diluted in the culture medium to a concentration of 2.0×10⁶ cells/mL or less. The cells were seeded in a culture bag so that the ratio of suspension to MACS GMP T-Cell TransACT (Miltenyi Biotec) was 17.5:1, and cultured for 36 to 48 hours (activation step). The activated cells were diluted in the culture medium using the LOVO Cell Processing System (Fresenius Kabi), seeded at a concentration of 6.07×10⁵ cells/cm² or less in a culture bag that was pre-coated with Retronectin (Takara Bio Inc.) and Retrovirus, and cultured until the next day (gene transduction step). In the gene transduction step, an exogenous gene that comprises a CAR gene (comprising anti-GPC3 scFv, CD8 hinge region, CD8 transmembrane region, CD28 intracellular region, 4-1BB intracellular region, and CD3ζ intracellular region), IL-7 gene, and CCL19 gene, wherein those genes were each connected through a nucleotide sequence encoding self-cleaving peptide (2A peptide), was introduced. The cells were seeded at a concentration of 2.2×10⁶ cells/cm² or less in a culture bottle (G-REX, Wilson Wolf), and cultured for 3 to 7 days.

### (4) Flow Cytometry

The percentage of CAR-expressing cells and the number of CAR-expressing cells in the samples after production were calculated by measuring the CAR-expressing cells in the CD45-expressing or CD3-expressing cells in the samples using Protein-L. The percentage of cells expressing T-cell activation markers (CD25 and CD69) immediately after the activation step was calculated by measuring the cells expressing these markers in the CD3-expressing cells or CD4 or CD8 expressing cells in the samples.

### (5) Analysis of Residual Amounts of TransACT and IL-2 Used for Activation

The residual amount of TransACT in the medium after the activation step was measured using T Cell Activation Bioassays (Promega, J1621). The residual amount of IL-2 was measured using Human IL-2 DuoSet ELISA (R & D systems, cat # DY202) .

### (II) Results

Fig. 1 shows the results in which the percentage of CAR-expressing cells and the number of CAR-expressing cells in the cell populations (activation step of 24, 48, or 72 hours) obtained according to "(2) Production of CAR-T Cells on Small Scale Using PBMC" were measured by flow cytometry. The percentage of CAR-expressing cells became the highest at 48 hours in a donor-independent manner (Fig. 1A). There was also a tendency for the number of CAR-expressing cells to become high at 48 hours (Fig. 1B) .

Fig. 2 shows the results in which the percentage of CAR-expressing cells and the number of CAR-expressing cells in the cell populations (activation step of 40, 44, or 48 hours) obtained according to "(2) Production of CAR-T Cells on Small Scale Using PBMC" were measured by flow cytometry. The percentage of CAR-expressing cells became the highest at 40 hours in a donor-independent manner (Fig. 2A). There was also a tendency for the number of CAR-expressing cells to become high at 40 hours (Fig. 2B) .

Fig. 3 shows the results in which the percentage of CAR-expressing cells in the cell populations (activation step of 37 to 44 hours) obtained according to "(2) Production of CAR-T Cells on Small Scale Using PBMC" was measured by flow cytometry. The CAR-T cells were produced with high efficiency at an activation duration of 37 hours or more and 44 hours or less.

Fig. 4 shows the results in which activated T cells in the cell populations (activation step of 40, 44, or 48 hours) obtained according to "(2) Production of CAR-T Cells on Small Scale Using PBMC" were measured by flow cytometry using an activation marker (CD25 or CD69) as an index, and the results in which the amounts of TransACT and IL-2 used for activation remaining in culture supernatants were analyzed. The percentage of CD25-expressing or CD69-expressing cells did not differ between the activation durations (40, 44, and 48 hours) immediately after the activation step (Fig. 4A and B). The amounts of TransACT and IL-2 used for activation remaining in the culture supernatants immediately after the activation step did not differ between the activation durations (40, 44, and 48 hours) (Fig. 4C and D). The production efficiency of CAR-T cells shown in the results of Figs. 1 to 3 is considered to have increased due to a factor other than the degree of activation and the main component in the medium.

Fig. 5 shows the results in which the percentage of CAR-expressing cells in cell populations (activation step of 36, 40, 44, or 48 hours) obtained according to "(3) Production of CAR-T Cells on Clinical Scale Using T Cells" in the Examples were measured by flow cytometry. The percentage of CAR-expressing cells tended to be high at 40 hours in a donor-independent manner. The percentage of CAR-expressing cells decreased at 36 hours, and the activation for 36 hours was insufficient for the production of CAR-T cells.

## Claims

1. A method for activating T cells comprising the step of activating T cells in a medium containing a CD3 agonist and/or a CD28 agonist for more than 36 hours and less than 48 hours.

2. The activation method according to claim 1, wherein the CD3 agonist and/or CD28 agonist is supported on a carrier.

3. The activation method according to claim 1, wherein the carrier is a matrix of mobile polymer chains or a bead.

4. The activation method according to claim 1, wherein the CD3 agonist and the CD28 agonist are an anti-CD3 antibody and an anti-CD28 antibody, respectively.

5. A method for producing genetically modified T cells, comprising the steps of:
activating T cells according to the activation method of claim 1,
introducing an exogenous gene into the activated T cells, and
culturing the T cells into which the exogenous gene has been introduced.

6. A cell population comprising genetically modified T cells obtained by the production method according to claim 5.

7. The production method according to claim 5, wherein the exogenous gene is a chimeric antigen receptor gene.

8. A matrix of mobile polymer chains or a bead supporting a CD3 agonist and/or a CD28 agonist, wherein the matrix or bead is used for being added to a medium and subjecting T cells to an activation step for more than 36 hours and less than 48 hours.

9. A T-cell activation kit for introducing an exogenous gene, comprising
a CD3 agonist and/or a CD28 agonist supported on a carrier, and
a manual describing the activation of T cells in a medium comprising the CD3 agonist and/or the CD28 agonist for more than 36 hours and less than 48 hours.
